# EUROPÄISCHE PATENTSCHRIFT

(11) **EP 2 398 379 B1**
(45) Veröffentlichungstag und Bekanntmachung des Hinweises auf die Patenterteilung: **01.05.2013**
(21) Anmeldenummer: 10711026.4
(22) Anmeldetag: 23.02.2010
(51) Int. Cl.: A61B 5/00, A61B 5/107, G01B 11/24

(54) **HANDGEHALTENE DENTALE KAMERA UND VERFAHREN ZUR OPTISCHEN 3D-VERMESSUNG**
HANDHELD DENTAL CAMERA AND METHOD FOR OPTICAL 3D MEASUREMENT
CAMÉRA DENTAIRE TENUE MANUELLEMENT ET PROCÉDÉ DE RELEVÉ OPTIQUE TRIDIMENSIONNEL

(30) Priorität: 23.02.2009 DE 102009001086
(43) Veröffentlichungstag der Anmeldung: 28.12.2011
(73) Patentinhaber: Sirona Dental Systems GmbH, 64625 Bensheim (DE)
(72) Erfinder: THIEL, Frank, 64372 Ober-Ramstadt (DE)
(74) Vertreter: Sommer, Peter
(86) Internationale Anmeldenummer: PCT/EP2010/052241
(87) Internationale Veröffentlichungsnummer: WO 2010/094805

(56) Entgegenhaltungen:
- WO-A2-2005/116578
- DE-A1-102007 005 625
- DE-A1-102007 005 726
- US-A- 6 019 721

## Beschreibung

### Technisches Gebiet

Die Erfindung betrifft eine handgehaltene dentale Kamera zur optischen 3D-Vermessung unter Verwendung einer konfokalen Messmethode, die ein chromatisches Objektiv, eine polychromatische Lichtquelle und einen Farbsensor umfasst und ein Verfahren zur Verwendung der erfindungsmäßigen dentalen Kamera.

### Stand der Technik

Aus dem Stand der Technik ist die konfokale Mikroskopie bekannt und wird unter anderem in der Patentschrift US 3,013,467 offenbart.

Die chromatische konfokale Messmethode stellt eine Möglichkeit dar, eine Durchfokussierung ohne mechanisch bewegte Teile zu realisieren und damit die Messzeit in der Regel deutlich zu verringern, welche von G. Molesini 1983 in Verbindung mit einem Spektrometer vorgeschlagen wurde (GB 2144537 und DE 3428593 C2). Ein Beispiel für eine erfolgreiche Applikation der chromatischen konfokalen Messmethode wird von H.J. Tiziani und H.-M. Uhde im Fachartikel "Three-dimensional image sensing by chromatic confocal microscopy" in Applied Optics, Vol. 33, No. 1, April 1994, auf den Seiten 1838 bis 1843 dargestellt. Die spektrale Analyse erfolgt hierbei mit drei Farbfiltern. Damit sind der erreichbare Tiefenmessbereich und die Tiefenauflösung jedoch begrenzt.

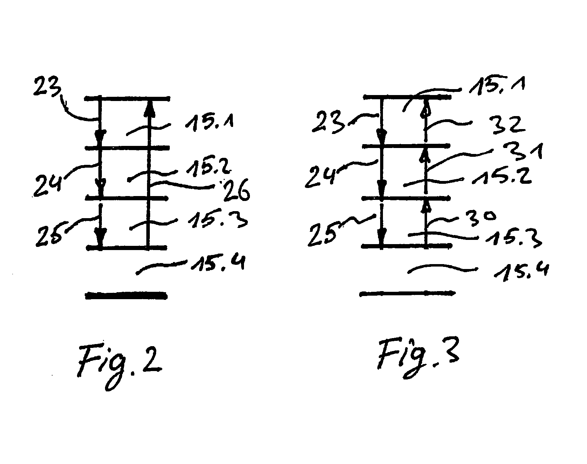

In der Patentschrift DE 103 21 885 A1 wird eine chromatische konfokale Anordnung mit einer brechkraftvariablen Komponente, beispielsweise mit einer diffraktiven Komponente, offenbart. Die in Figur 2 dieser Patentschrift dargestellte optische Anordnung ist zur Gewinnung der konfokalen Signale über der Wellenlänge zur Beleuchtung einer Reihe von Mikrolinsen angeordnet und zur Analyse ein Spektrometer mit einer Flächenkamera nachgeordnet, so dass Linienprofile in einem einzigen flächigen Kamerabild mittels eines Linien-Spektrometers aufgenommen werden können. In der Veröffentlichung "Chromatic confocal detection for speed microtopography measurements" von A. K. Ruprecht, K. Körner, T.F. Wiesendanger, H.J. Tiziani, W. Osten in Proceedings of SPIE, Vol. 5302-6, S. 53-60, 2004 wird in der Abbildung 4 ein chromatischer konfokaler Liniensensor zur linienhaften Topografiemessung offenbart. Dort ist zur Gewinnung der konfokalen Signale über der Wellenlänge der chromatischen konfokalen Anordnung ein Linien-Spektrometer nachgeordnet, so dass Linienprofile von einer Objektoberfläche in einem einzigen Kamerabild unter Einsatz einer einzigen Flächenkamera und eines Linien-Spektrometers aufgenommen werden können. Der Einsatz eines Spektrometers gestattet grundsätzlich eine höhere spektrale Auflösung im Vergleich zu einer Anordnung mit drei Farbfiltern oder einer RGB-Farbkamera oder auch einer Vierkanal-Farbkamera und ist damit vorteilhafter.

In der Dissertation von J. Schmoll mit dem Titel "3D-Spektrofotometrie extragalaktischer Emissionslinien", eingereicht bei der Universität Potsdam im Juni 2001, wird auf den Seiten 12 bis 13 die Linsenraster-Direktankopplung beschrieben, die von Courtes et al. erstmals 1988 beim TIGER-Spektrografen eingesetzt wurde. Hierbei wird das Linsenraster um einen Winkel gegen die Dispersionsrichtung verdreht. Diese Technik gilt in der Auswertung durch die Verschiebung benachbarter Spektren als kompliziert und der Flächensensor wird in seiner Fläche nicht ökonomisch ausgenutzt, da der Flächenfüllfaktor gering ist. In wissenschaftlichen Arbeiten werden hier auch die Begriffe 3D-Spektrofotometrie und abbildende Spektroskopie sowie auch Integral-Field-Spektrofotometrie verwendet.

In der DE 10 2007 005 726 A1 das die Merkmale des Oberbegriffs des Anspruch 1 offenbart, wird eine handgehaltene dentale Kamera zur optischen 3-D-Vermessung mit einem chromatischen Objektiv und einer polychromatischen Lichtquelle beschriben. Durch das Verschieben einer Scanneinheit wird ein breiter chromatischer horizontalen Messbereich abgetastet.

Die chromatische konfokale Messmethode hat den Vorteil, dass die Kamera im Prinzip ohne mechanisch bewegte Teile realisiert werden kann und dass die Datenrate gering ist, da man für einen Messpunkt nur ein einziges Farbspektrum aufnehmen muss.

Die chromatische konfokale Messmethode hat jedoch den Nachteil, dass eine spektralbreitbandige Lichtquelle verwendet werden muss, die ein möglichst breites und kontinuierliches Wellenlängenspektrum aufweist. Als Lichtquellen sind daher vor allem Halogen- und Xenon-Gasentladungslampen geeignet. Diese Lichtquellen sind bauartbedingt vergleichsweise unhandlich und groß. Eine kompakte Lichtquelle, wie eine Laserdiode oder eine Superlumineszenzdiode ist für die chromatische konfokale Messmethode weniger geeignet, da sie typischerweise ein eher schmales Wellenlängenspektrum aufweist. Der Tiefenmessbereich ist daher stark eingeschränkt und für die Vermessung von relativ großen Objekten, wie von Zähnen, nicht geeignet.

Bei einer klassischen scannenden konfokalen Messmethode mit einem mechanischen Tiefenscan wird die Position eines einigen Fokuspunktes durch mechanisches Verschieben einzelner Linsenelemente der Optik oder durch Verschieben der gesamten Optik relativ zum Objekt verschoben. Als Lichtquelle wird eine Lichtquelle mit einem möglichst schmalen Wellenspektrum gewählt, um die Ausdehnung des Fokuspunktes gering zu halten. Zur Vermessung eines einzelnen Messpunktes muss demnach die Optik schrittweise mechanische entlang der gesamten Messtiefe verschoben, für jede Position der Optik ein Datensatz aufgenommen und anschließen aus den gesamten ermittelten Datensätzen ein Höhenwert bestimmt werden. Die Auflösung der Höhenwerte hängt dabei von der Schrittweite der einzelnen mechanischen Verschiebe-Schritte der Optik ab. Daher hat die klassische scannende konfokale Messmethode den Nachteil, dass für eine gute Auflösung sehr hohe Datenmengen anfallen und verarbeitet werden müssen.

Die klassische scannende konfokale Messmethode hat dafür den Vorteil, dass sich kompakte Lichtquellen, wie LED und LD einsetzen lassen, die ein schmalbandiges Wellenlängenspektrum aufweisen.

Die Aufgabe dieser Erfindung besteht demnach darin, eine konfokale Vorrichtung bereitzustellen und ein konfokales Verfahren zu realisierten zu realisieren, welches eine schnelle optische 3D-Vermessung des Messobjekts ermöglicht, wobei eine kompakte Lichtquelle verwendet werden kann und wobei geringe Datenraten anfallen.

### Darstellung der Erfindung

Diese Aufgabe wird durch die erfindungsmäßige handgehaltene dentale Kamera gemäβ Anspruch 1 und das erfindungsmäßige Verfahren gemäβ Anspruch 11 gelöst.

Die erfindungsgemäße handgehaltene dentale Kamera zur optischen 3D-Vermessung umfasst ein chromatisches Objektiv, eine polychromatische Lichtquelle und einen Farbsensor, wobei die polychromatische Lichtquelle einen Beleuchtungsstrahl (8) abstrahlt, der mittels des chromatischen Objektivs auf eine Oberfläche eines Messobjekts zumindest bezüglich einer Wellenlänge fokussierbar ist. Der Beleuchtungsstrahl wird von der Oberfläche als ein Beobachtungsstrahl zurückgestrahlt und ist mittels des Farbsensors detektierbar. Die Fokuspunkte verschiedener Wellenlängen des Beleuchtungsstrahls bilden einen chromatischen Tiefenmessbereich.

Weiterhin weist die handgehaltene dentale Kamera eine verstellbare Scaneinheit auf, die zumindest das chromatische Objektiv umfasst. Der chromatische Tiefenmessbereich ist mittels der Scaneinheit schrittweise verschiebbar, so dass an einen ersten chromatischer Tiefenmessbereich in einer ersten Stellung der Scaneinheit ein zweiter chromatischen Tiefenmessbereich in einer zweiten Stellung der Scaneinheit anschließt oder sich mit dem ersten chromatischen Tiefenmessbereich teilweise überlappt. Auf diese Weise wird aus den zwei verschiedenen Tiefenmessbereichen ein vergrößerter, gesamter Tiefenmessbereich gebildet.

Die handgehaltene dentale Kamera zur optischen 3D-Vermessung kann eine handgehaltene Kamera sein, die insbesondere für dentale intraorale Aufnahmen von Zähnen geeignet ist und die Prinzipien der chromatisch konfokalen und der scannenden konfokalen Tiefenvermessung vereint.

Bei der chromatischen konfokalen Messmethode erfolgt die Vermessung ohne mechanische Verstellung der Optik, indem die Fokuspunkte unterschiedlichen Wellenlängen über die gesamte Messtiefe verteilt werden und unter Verwendung der spektralen Analyse diejenige Wellenlänge ermittelt wird, deren Fokuspunkt auf der Oberfläche liegt. Von dieser Wellenlänge kann dann auf die Fokuslage, also die z-Koordinate der Objektoberfläche geschlossen werden. Die Auflösung der z-Koordinate hängt vor allem von der kontinuierlichen Verteilung der Wellenlängen im Spektrum des verwendeten Beleuchtungsstrahls und der Genauigkeit der spektralen Analyse ab.

Dafür wird die polychromatische Lichtquelle verwendet, deren als Beleuchtungsstrahl abgestrahlter Spektralbereich mehrere Wellenlängen umfasst. Dieser Beleuchtungsstrahl wird mit einem chromatischen Objektiv auf das Messobjekt fokussiert. Da ein chromatisches Objektiv den Effekt der chromatischen Abberationen verstärkt, werden Fokuspunkte für die unterschiedlichen Wellenlängen des Beleuchtungsstrahls deutlich auseinandergezogen. Die Fokuspunkte der kürzesten und der längsten Wellenlänge des Spektralbereichs des Beleuchtungsstrahls können bis zu 5 mm auseinander liegen und bilden den chromatischen Tiefenmessbereich der handgehaltenen dentalen Kamera. Innerhalb dieses chromatischen Tiefenmessbereichs kann jeder Wellenlänge ein Höhenwert zugeordnet werden.

Durch dieses auseinanderziehen der Fokuspunkte liegt nur der Fokuspunkt einer einzelnen Wellenlänge oder zumindest eines sehr schmalen Wellenlängenbereichs des Spektralbereichs des Beleuchtungstrahls genau auf der Oberfläche des Messobjekts und dominiert damit das spektrale Intensitätsprofil des Beobachtungsstrahls.

Der Beobachtungsstrahl wird mit einem Farbsensor detektiert, der in der Lage ist einen breiten Spektralbereich aufzunehmen und die einzelnen Wellenlängen voneinander zu unterscheiden. Dazu ist insbesondere ein Spektrometer oder auch ein CCD-Sensor geeignet.

Damit kann die Wellenlänge mit maximaler Intensität des Beobachtungsstrahls ermittelt werden und dem Messpunkt der Oberfläche ein Höhenwert entsprechend dieser Wellenlänge zugeordnet werden, soweit der Messpunkt innerhalb des chromatischen Tiefenmessbereichs liegt.

Bei der vorliegenden Erfindung werden kompakte polychromatisches Lichtquellen, wie LED, Leserdioden (LD) und Superlumineszenzdioden (SLD), verwendet deren Wellenlängenspektrum im Vergleich zu Halogen- oder Xenon-Gasentladungslampen schmal ist. Um trotzdem einen ausreichend großen Tiefenmessbereich vermessen zu können, wird die chromatisch konfokale Messmethode mit der klassisch scannenden konfokalen Messmehtode kombiniert.

Bei der scannenden konfokalen Messmethode wird ein einzelner Fokuspunkt durch schrittweise mechanisches Verschieben der Optik entlang der Messtiefe verschoben, wobei aus den ermittelten Intensitäten des Beobachtungsstrahls ermittelt wird, bei welchem Schritt des mechanischen Verschiebens der Optik der Fokuspunkt genau auf der Oberfläche liegt. Von diesem Schritt, bei dem ein Maximum der Intensität des Beleuchtungsstrahls aufgetreten ist, kann dann auf die Fokuslage geschlossen werden. Die Auflösung der z-Koordinate, also des Höhenwertes, wird bei dieser Methode durch die Schrittweite des mechanischen Verschiebens der Optik bestimmt.

Mit der Scaneinheit der handgehaltenen dentalen Kamera, die zumindest das chromatische Objektiv umfasst, können die durch das Verwenden der polychromatischen Lichtquelle und des chromatischen Objektivs auseinandergezogenen mehreren Fokuspunkte gleichzeitig entlang der Messtiefe verschoben werden. Die Schrittweite kann so gewählt werden, dass sie möglichst genau der Länge des chromatischen Tiefenmessbereichs entspricht. Damit können mehrere chromatische Tiefenmessbereiche, die entlang der z-Achse aneinander anschließen oder überlappen, nacheinander vermessen werden und aus den ermittelten Datensätzen ein 3D-Datensatz für einen vergrößerten gesamten Tiefenmessbereich ermittelt werden. Dieser vergrößerte Tiefenmessbereich ergibt sich aus der Summe der aneinander anschließenden oder überlappenden chromatischen Tiefenmessbereiche.

Beispielsweise kann der chromatische Tiefenmessbereich, der sich mit einer kompakten Lichtquelle, wie einer LED, LD oder SLD erreichen lässt, 0,5mm betragen. Soll ein gesamter Tiefenmessbereich von 20mm vermessen werden, so kann dies in 40 Schritten mit einer Schrittweite von 0,5mm geschehen.

Trotz eines schmalen chromatischem Tiefenmessbereichs kann so eine Messtiefe erreicht werden, die das Vermessen eines Objekts, wie eines Zahns, ermöglicht.

Ein Vorteil gegenüber der rein chromatisch konfokalen Methode ist, dass kompaktere Lichtquellen, wie LED, LD und SLD, verwendet werden können, da bereits ein geringer Spektralbereich Δλ ausreichend ist. Auf die unhandlichen und großen Lichtquellen, wie die Halogen- und Xenon- Gasentladungslampen, die typischerweise bei der chromatischen konfokalen Messmethode verwendet werden, kann daher verzichtet werden.

Ein weiterer Vorteil der erfinderischen handgehaltenen dentalen Kamera ist, dass die Anzahl der nötigen mechanischen Schritte der Scaneinheit im Vergleich zur rein scannenden konfokalen Methode deutlich reduziert und damit auch die zu verarbeitende Datenmenge deutlich verringert wird.

Vorteilhafterweise kann die Scaneinheit in genau zwei Stellungen gebracht werden, nämlich von der einen Endstellung unmittelbar in eine andere Endstellung.

Dadurch kann ein doppelt so großer Tiefenmessbereich vermessen werden, als es bei der rein chromatisch konfokalen Methode möglich wäre und das mechanische Verschieben ist gegenüber der klassisch scannenden konfokalen Methode vereinfacht, da von der Scaneinheit nur zwei Positionen angefahren werden, die jeweils mit einem Endanschlag realisiert werden können.

Vorteilhafterweise umfasst die handgehaltene dentale Kamera ein Umlenkmittel, wobei das Umlenkmittel zwischen dem chromatischen Objektiv und dem Messobjekt angeordnet ist, wobei der Beleuchtungsstrahl mittels des Umlenkmittels zum Messobjekt hin quer zur Längsachse der handgehaltenen dentalen Kamera umlenkbar ist.

Das Umlenkmittel kann ein Prisma oder ein Spiegel, der mit einem festen Winkel von 45° zum Beleuchtungsstrahl angeordnet ist, sein, so dass der Beleuchtungsstrahl in einem Winkel von 90° zum Messobjekt umgelenkt wird. Dadurch kann die erfinderische handgehaltene dentale Kamera sehr kompakt gebaut werden und intraorale Aufnahmen aus einer schwer zugänglichen Richtung in der Mundhöhle des Patienten ermöglichen.

Vorteilhafterweise kann die Lichtquelle eine Halogen- oder Xenon-Gasentladungslampe mit einem Spektrum der Wellenlänge zwischen 500 nm und 2000 nm sein.

Dadurch wird ein breites Wellenlängenspektrum bereitgestellt, so dass der chromatische Tiefenmessbereich größer ist und der erforderliche gesamte Tiefenmessbereich in nur wenigen Schritten abgedeckt werden kann. Da Halogen- und Xenon-Gasentladungslampen allerdings zu groß sind, um sie in einer kompakten handgehaltenen dentalen Kamera zu integrieren, können sie beispielsweise über einen Lichtleiter an die handgehaltene dentale Kamera angeschlossen werden.

Vorteilhafterweise kann die Lichtquelle eine Superlumineszenzdiode (SLD) sein.

Eine Superlumineszenzdiode (SLD) ist eine Diode mit einem relativ breiten Wellenlängenspektrum, die gegenüber Laserdioden vergleichbare Ausgangsleistungen bei einem äußerst geringen spektralen Rauschen aufweist.

Die Verwendung einer SLD hat den Vorteil, dass die SLD aufgrund ihrer kompakten Bauweise in eine handgehaltene dentale Kamera integrierbar ist und trotz der Kompaktheit ein relativ breites Wellenlängenspektrum bereitgestellt wird.

Vorteilhafterweise kann die Superlumineszenzdiode (SLD) ein Spektrum mit einer Wellenlänge zwischen 900 nm und 1000 nm aufweisen.

Es gibt verschiedene Arten von Superlumineszenzdioden mit unterschiedlichen Wellenlängenbereichen. Die SLD mit einem Wellenlängenbereich zwischen 900nm und 1000nm weist jedoch eine relativ konstante Intensität bezogen auf die Wellenlängen auf. Der Farbsensor muss dabei dem wellenlängenabhängigen Intensitätsprofil entsprechend so gewählt werden, dass die wellenlängenabhängige Nachweiseffizienz nicht gegen Null geht.

Vorteilhafterweise kann die Superlumineszenzdiode (SLD) ein Spektrum mit einer Wellenlänge zwischen 1500 nm und 1650 nm aufweist.

Dadurch wird ein breiteres Wellenlängenspektrum bereitgestellt, so dass die Anzahl der mechanischen Scanschritte reduziert werden kann.

Vorteilhafterweise kann die Lichtquelle eine weisslicht LED sein.

Aufgrund der Kokmpaktheit einer weisslicht LED ist es möglich diese in handgehaltene dentale Kamera zu integrieren und das breitere Spektrum einer weisslicht LED ermöglicht es die Anzahl der mechanischen Scanschritte zu reduzieren. Vorteilhafterweise kann der chromatische Tiefenmessbereich eine Länge zwischen 0,5 mm und 5 mm aufweisen.

Dadurch ist die erfinderische handgehaltene dentale Kamera besonders gut für die Vermessung von Zähnen geeignet. Dadurch kann beispielsweise ein gesamter Tiefenmessbereich von 30 mm in lediglich sechs Schritten von jeweils 5 mm vermessen werden.

Vorteilhafterweise weist die handgehaltene dentale Kamera eine Datenverarbeitungseinheit oder einen Anschluss auf, an den eine Datenverarbeitungseinheit anschließbar ist. Mit der Datenverarbeitungseinheit kann für jeden chromatischen Tiefenmessbereichen ein Datensatz aufgenommen und erzeugt werden. Diese verschiedenen Datensätze können dann zu einem gesamten 3D-Datensatz über den gesamten Tiefenmessbereich zusammenfügt werden.

Dadurch kann die handgehaltene dentale Kamera in einer kompakten Bauweise ausgeführt sein und ein Tiefenmessbereich abgedeckt werden, der es ermöglicht ganze Zähne zu vermessen.

Vorteilhafterweise ist die Scaneinheit mit einer Frequenz zwischen 1Hz und 1000Hz mechanische verstellbar.

Dadurch kann die Oberfläche des Messobjekts in relativ kurzer Zeit vermessen werden. Bei einer handgehaltenen Kamera ist es besonders wichtig, dass das Aufnahmezeitintervall möglichst kurz ist, da ein Benutzer die Kamera nur für sehr kurze Intervalle wirklich ruhig halten kann.

Vorteilhafterweise ist ein schwenkbarer Spiegel zwischen der Lichtquelle und der Oberfläche des Objekts angeordnet, so dass durch ein schrittweises Verkippen dieses schwenkbaren Spiegels der Beleuchtungsstrahl schrittweise in lateraler Richtung über die gesamte Oberfläche des Messobjekts bewegt werden kann.

Je nach Ausführungsform ist als Beleuchtungsstrahl ein Array, welches die Oberfläche des Messobjekts insgesamt abdeckt, eine Zeile oder ein einzelner Punkt in der xy-Ebene vorgesehen. Handelt es sich um eine Zeile, so muss diese in der Richtung senkrecht zur Zeile schrittweise über das Objekt bewegt und jeweils eine Aufnahme gemacht werden, so dass aus den einzelnen Datensätzen ein Datensatz für das gesamte Messobjekt zusammengesetzt werden kann. Ein Punkt muss entsprechend in x- und in y-Richtung schrittweise über das Objekt bewegt werden und ein gesamter Datensatz des Messobjekts aus den einzelnen Datensätzen zusammengesetzt werden.

Ein weiterer Gegenstand der Erfindung ist ein Verfahren zur optischen 3D-Vermessung, bei dem ein Beleuchtungsstrahl einer polychromatischen Lichtquelle, der durch ein chromatisches Objektiv auf eine Oberfläche eines Messobjekts zumindest bezüglich einer Wellenlänge fokussiert wird und der von der Oberfläche als ein Beobachtungsstrahl zurückgestrahlte Beleuchtungsstrahl mit einem Farbsensor detektiert wird. Dabei bilden die Fokuspunkte verschiedener Wellenlängen des Beleuchtungsstrahls einen chromatischen Tiefenmessbereich. Eine Scaneinheit, die zumindest das chromatische Objektiv umfasst, wird schrittweise verschoben, so dass an einen erster chromatischer Tiefenmessbereich in einer ersten Stellung der Scaneinheit mindestens ein zweiter chromatischen Tiefenmessbereich in einer zweiten Stellung der Scaneinheit angeschlossen wird oder mit dem ersten chromatischen Tiefenmessbereich teilweise überlappt wird. Auf diese Weise wird aus den mindestens zwei Tiefenmessbereichen ein vergrößerter, gesamter Tiefenmessbereich gebildet.

Ein Vorteil des erfinderischen Verfahrens ist, dass die Vermessung im Vergleich zum klassischen scannenden Verfahren schneller erfolgt, da die Scaneinheit nicht kontinuierlich, sondern nur zwischen einigen mindestens zwei festgestellten Stellungen mechanisch verstellt wird.

Ein weiterer Vorteil des erfinderischen Verfahrens ist, dass geringere Datenraten anfallen, die unter Verwendung der vorhandenen Methoden in einer relativ kurzen Zeit analysiert werden können.

Vorteilhafterweise wird die Scaneinheit genau einmal von einer Endstellung unmittelbar in eine andere Endstellung verschoben.

Dadurch kann ein größerer Tiefenmessbereich vermessen werden, als es bei der rein chromatischen konfokalen Methode möglich wäre und das Verschieben der Scaneinheit kann einfacher erfolgen, als es bei der klassischen scannenden konfokalen Methode möglich ist, da nur zwei Positionen angefahren werden, die entsprechend jeweils mit einem Endanschlag versehen werden können.

Vorteilhafterweise wird der Beleuchtungsstrahl mittels eines Umlenkmittels zum Messobjekt umgelenkt.

Dadurch wird der Beleuchtungsstrahl in seiner Richtung auf eine einfache Weise beispielsweise mittels eines Umlenkspiegels verstellt.

Vorteilhafterweise wird eine Lichtquelle mit eine Spektralbereich von 300nm bis 2000nm verwendet.

Dadurch kann in einem einzelnen Schritt ein relativ großer chromatischer Tiefenmessbereich abgetastet werden, so dass zur Vermessung des gesamten Objekts nur wenige Schritte notwendig sind.

Vorteilhafterweise wird eine Lichtquelle mit eine Spektralbereich von 900nm bis 1000nm oder von 1500nm bis 1650nm verwendet.

Dadurch werden zwar mehr Schritte zur Vermessung des gesamten Projekts benötigten, es können jedoch kompakte Lichtquellen, wie LED, LD und SLD, verwendet werden.

Vorteilhafterweise werden Fokuspunkte für verschiedene Wellenlängen aufgefächert, so dass sich ein Länge des chromatischen Tiefenmessbereichs zwischen 0,5mm und 5mm ergibt.

Dadurch werden weniger Verschiebe-Schritte der Scaneinheit benötigt, was ein schnelleres Vermessen des Messobjekts ermöglicht.

Vorteilhafterweise wird für jeden chromatischen Tiefenmessbereich ein Datensatz aufgenommen wird, wobei die Datensätze innerhalb der handgehaltenen dentalen Kamera gespeichert und zu einem 3D-Datensatz des Messobjekts zusammengefügt oder an eine Datenverarbeitungseinheit übermittelt werden.

Sind nur geringe Datenmengen zu verarbeiten können durch eine verarbeitung dieser Daten innerhalb der Kamera Übertragungszeiten eingespart werden. Bei größeren Datenmengen kann es vorteilhaft sein, die Datenverarbeitung außerhalb der Kamera vorzunehmen.

Vorteilhafterweise wird die Scaneinheit mit einer Frequenz zwischen 1 Hz und 1000 Hz verschoben.

Dadurch kann die Oberfläche des Messobjekts in relativ kurzer Zeit vermessen werden und eine entsprechende handgehaltene dentale Kamera kann daher von Hand gehalten werden, um eine Aufnahme zu machen.

Vorteilhafterweise wird der Beleuchtungsstrahl durch schrittweises Verkippen eines schwenkbaren Spiegels in lateraler Richtung über die gesamte Oberfläche des Messobjekts bewegt.

Dadurch kann das gesamte Messobjekt erfasst werden, auch wenn der Beleuchtungsstrahl nur punkt- oder zeilenförmig auf das Objekt auftrifft.

Vorteilhafterweise erfolgt das Verschieben der Scaneinheit zum Vermessen der einzelnen chromatischen Messbereiche, die den gesamten Messbereich ergeben, in mehreren Schritten. Anschließend wird dann die Scaneinheit in einem weiteren Schritt wieder in die erste Stellung gebracht, wobei dieser Messzyklus der Scaneinheit solange wiederholt wird, bis die Messung abgeschlossen ist.

Dadurch wird der Beleuchtungsstrahl schrittweise von einem oberen Ende des gesamten Tiefenmessbereichs bis zu einem unteren Ende des gesamten Tiefenmessbereichs verstellt werden und zur Vermessung des nächsten benachbarten Messpunktes wieder in die Ausgangsstellung am oberen Ende des gesamten Tiefenmessbereichs gebracht werden. Dieser Messzyklus ermöglicht eine schnellere Vermessung der gesamten Oberfläche.

Vorteilhafterweise erfolgt das Verschieben der Scaneinheit zum Vermessen der einzelnen chromatischen Messbereiche, die den gesamten Messbereich ergeben, in mehreren Schritten. Anschließend wird die Scaneinheit wieder in Schritten in die erste Stellung gebracht, wobei dieser Messzyklus der Scaneinheit solange wiederholt wird, bis die Messung abgeschlossen ist.

Durch diesen Messzyklus wird der Schritt, bei denen der Beleuchtungsstrahl wieder in die Ausgangsstellung verstellt wird, eingespart, so dass die Dauer der Messung verkürzt wird.

Kurzbeschreibung der Zeichnungen Ausführungsbeispiele der Erfindung sind in der Zeichnung dargestellt. Es zeigt die
- Fig. 1: eine handgehaltene dentale Kamera;
- Fig. 2: ein erstes Schema einer Messsequenz
- Fig. 3: eine zweites Schema einer Messsequenz. Ausführungsbeispiel

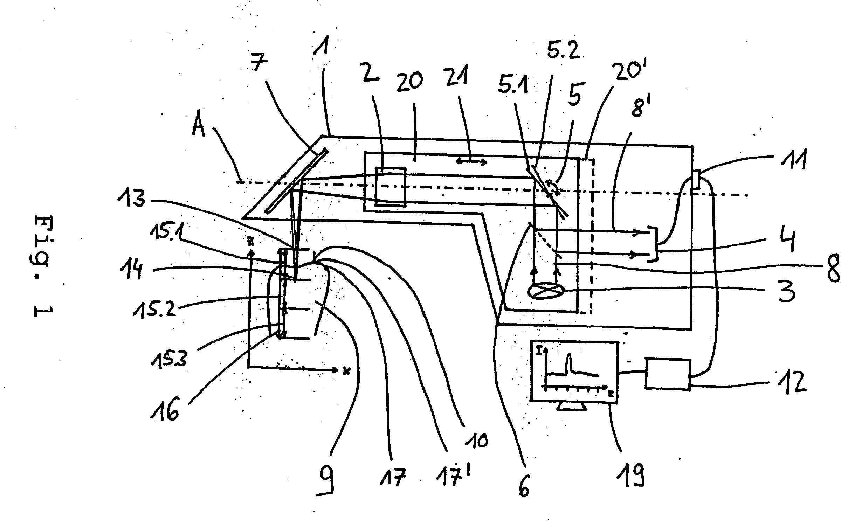

Die Fig. 1 zeigt ein Ausführungsbeispiel einer erfindungsmäßigen handgehaltenen dentalen Kamera 1 zur 3D-Vermessung. Die handgehaltene dentale Kamera 1 umfasst eine Scaneinheit, welche in diesem Ausführungsbeispiel aus einem chromatisches Objektiv 2, eine polychromatische Lichtquelle 3, einem schwenkbaren Spiegel 5 und einem Strahlteiler 6 besteht und innerhalb der handgehaltenen dentalen Kamera 1 entlang der Längsachse A verschiebbar ist. Weiterhin umfasst die handgehaltene dentale Kamera 1 einen Farbsensor 4, ein Umlenkmittel 7, beispielsweise einen Umlenkspiegel, und einen Anschluss 11, an dem man eine Datenverarbeitungseinheit 12 anschließen kann.

Die polychromatische Lichtquelle 3 sendet einen Beleuchtungsstrahl 8 aus, der den Strahlteiler 6, beispielsweise ein halbdurchlässiger Spiegel oder ein Strahlteiler Prisma, möglichst ungehindert durchläuft und vom schwenkbaren Spiegel 5 in Richtung des chromatischen Objektivs 2 umgelenkt wird. Der Beleuchtungsstrahl 8 wird von dem chromatische Objektiv fokussiert und von dem Umlenkmittel 7 zum Messobjekt 9 hin, beispielsweise ein Zahn, umgelenkt. Die Oberfläche 10 des Messobjekts 9 reflektiert einen Teil des Beleuchtungsstrahls 8, der als Beobachtungsstrahl 8' in die handgehaltene dentale Kamera 1 zurückgestrahlt wird. Der Beobachtungsstrahl 8' wird von dem Umlenkmittel 7 zum chromatischen Objektiv 2 umgelenkt, durchläuft das chromatische Objektiv 2, wird vom schwenkbaren Spiegel 5 zum Strahlteiler 6 umgelenkt und wird möglichst vollständig durch den Strahlteiler 6 zum Farbsensor 4, beispielsweise ein CCD-Sensor, umgelenkt. Die vom CCD-Sensor aufgenommenen Bilddaten werden über den Anschluss 11 an eine Datenverarbeitungseinheit 12 in Form eines PCs weitergeleitet.

Das erfinderische Verfahren der optischen 3D-Vermessung weist sowohl Elemente einer chromatischen konfokalen Messmethode als auch Elemente einer scannenden konfokalen Messmethode auf.

Die polychromatische Lichtquelle 3 weist einen Spektralbereich aus mehreren mindestens zwei Wellenlängen auf. Der von der Lichtquelle 3 abgestrahlte Beleuchtungsstrahl umfasst also mindestens zwei von einander verschiedene Wellenlängen. Als polychromatische Lichtquelle für die erfinderische handgehaltene dentale Kamera 1 kommt vor allem eine kompakte polychromatische Lichtquelle, wie eine LED, eine Laserdiode (LD) oder eine Superlumineszenzdiode (SLD) in Frage. Als Lichtquelle können auch Halogen- oder Xenon-Gasentladungslampen verwendet werden, die jedoch wegen ihrer Größe für die erfinderische handgehaltene dentale Kamera 1 nur geeignet sind, wenn sie nicht in der Kamera 1 selbst angeordnet, sondern über einen Lichtleiter mit dieser verbunden sind.

Der Strahlteiler 6 ist ein halbdurchlässiges optisches Element, das den Beleuchtungsstrahl zumindest teilweise ungehindert durchlässt und den Beobachtungsstrahl zur Detektion zumindest teilweise auf den Farbsensor umlenkt.

Der schwenkbare Spiegel 5 ist ein Spiegel der um mindestens eine Achse drehbar gelagert ist und mittels eines Elektromotors computergesteuert in seinem Winkel verstellt wird.

Durch das Verkippen des Spiegels in verschiedene Stellungen 5.1, 5.2 kann der Beleuchtungsstrahl 8 über das gesamte Messobjekt 9 bewegt werden, um für alle Punkte 17, 17' in der xy-Ebene des Messobjekts 9 einen Höhenwert, also eine z-Wert zu ermitteln und zu einem 3D-Datensatz des Messobjekts 9 zusammensetzen zu können.

Das chromatische Objektiv 2 ist ein optisches Element, das den Effekt der chromatischen Aberration verstärkt, so dass die Fokuspunkte für die unterschiedlichen Wellenlängen des Beleuchtungsstrahls 8 deutlich auseinandergezogen werden. Dadurch ergibt sich ein chromatischer Tiefenmessbereich 15.1, der sich von der Position eines ersten Fokuspunkts 13, der dem chromatischen Objektiv 2 am nächsten ist, bis zu der Position eines zweiten Fokuspunkts 14, der von dem chromatischen Objektiv 2 am weitesten entfernt ist, erstreckt. Innerhalb dieses chromatischen Tiefenmessbereichs 15.1 kann ein Höhenwert ermittelt werden. Die Messgenauigkeit hängt von dem Abstand der dazwischen liegenden Fokuspunkte der weiteren im Beleuchtungsstrahl 8 vorkommenden Wellenlängen ab.

Die Scaneinheit 20 ist eine Einheit, die beispielsweise mittels eines elektronisch gesteuerten Elektromotors innerhalb der handgehaltenen dentalen Kamera 1 entsprechend dem Pfeil 21 entlang der Längsachse A der handgehaltenen dentalen Kamera 1 verschoben wird, so dass sich die Fokuspunkte 13, 14 des Beleuchtungsstrahls 8 entlang der z-Achse, also entlang des Tiefenmessbereichs 15.1 quer zur Längsachse A der handgehaltenen dentalen Kamera 1, verschieben. So kann nach dem Vermessen eines ersten Tiefenmessbereichs 15.1 in einer ersten Stellung der Scaneinheit die Scaneinheit in eine zweite Stellung 20' verschoben werden, wodurch sich die Fokuspunkte 13, 14 soweit verschieben, dass die Position des Fokuspunkt 13 in der zweiten Stellung der Scaneinheit mit der Position des Fokuspunkts 14 in der ersten Stellung der Scaneinheit übereinstimmt. So schließt der Tiefenmessbereich 15.2 der zweiten Stellung der Scaneinheit an den Tiefenmessbereich der ersten Stellung der Scaneinheit an. Entsprechend können weitere Tiefenmessbereiche 15.3 an den Tiefenmessbereich 15.2 angeschlossen werden, in dem die Scaneinheit in weitere Stellungen entlang einer Längsache A der handgehaltenen dentalen Kamera 1 verschoben wird.

Der Farbsensor 4 ist ein lichtempfindlicher Sensor, der in der Lage ist ein Wellenlängenspektrum aufzunehmen und die Intensität der einzelnen Wellenlänge wiederzugeben. Dadurch kann ein wellenlängenabhängiges Intensitätsprofil des Beobachtungsstrahls 8 und damit die Wellenlänge mit der maximalen Intensität ermittelt werden, aus der sich der Höhenwert, also der z-Wert, ergibt. In der dargestellten Ausführungsform ist als Farbsensor ein CCD-Sensor vorgesehen, der Farbsensor könnte aber auch als ein Spektrometer ausgeführt sein. Der Farbsensor 4 ist über einen Anschluss 11 an eine Datenverarbeitungseinheit 12, beispielsweise einen Computer und eine Ausgabeeinheit 19 angeschlossen. So können die in verschiedenen Stellungen der Scaneinheit 20 für verschiedene chromatischen Tiefenmessbereich 15.1-4 nacheinander ermittelten Datensätze gespeichert und zu einem gesamten 3D-Datensatz des Messobjekts über den gesamten Tiefenmessbereich 16 zusammengefügt werden.

Die Fig. 2 zeigt eine schematische Skizze zur Verdeutlichung eines Verschiebezykluses der Scaneinheit 20 in mehreren Schritten. Nach der Aufnahme des ersten chromatischen Messbereichs 15.1 werden die Fokuspunkte zwischen den äußeren Fokuspunkten 13 und 14 in einem ersten Schritt 23 zum zweiten chromatischen Tiefenmessbereich 15.2 verschoben. Nach der Aufnahme des zweiten chromatischen Tiefenmessbereichs 15.2 werden die Fokuspunkte in einem zweiten Schritt 24 zum dritten chromatischen Tiefenmessbereich 15.3 verschoben. Nach der Aufnahme des dritten chromatischen Tiefenmessbereichs 15.3 werden die Fokuspunkte in einem dritten Schritt 25 zum vierten chromatischen Tiefenmessbereich 15.4 verschoben. In einem vierten Schritt 26 wird die Scaneinheit 20 wieder in ihre ursprüngliche Position gebracht, so dass die Fokuspunkte 13 und 14 wieder den ersten chromatischen Tiefenmessbereich 15.1 einschliessen.

Typischerweise wird zwischen den einzelnen Schritten des beschriebenen Messzykluses für den jeweilig in z-Richtung eingestellten Tiefenmessbereich 15.1, 15.2, 15.3, 15.4 der gesamte zu vermessende Bereich der xy-Ebene abgescannt, indem der Beleuchtungsstrahl durch schrittweises Verkippen des schwenkbaren Spiegels 5 schrittweise in x- und/oder y-Richtung versetzt wird. Der beschriebene Zyklus zur Verschiebung des chromatischen Tiefenmessbereichs wird also typischerweise zum Vermessen eines Objekts nur einmal durchlaufen.

Es besteht aber auch die Möglichkeit für jeden zu vermessenden Punkt in der xy-Ebene, also für jede Stellung 5.1, 5.2 des schwenkbaren Spiegels 5, den beschriebenen Messzyklus einmal zu durchlaufen.

Wird diese Variante gewählt, so kann der beschriebene Messzyklus auch dahingehen verändert werden, dass, wie in Fig. 3 als Skizze zur Verdeutlichung eines alternativen Verschiebezykluses der Scaneinheit in mehreren Schritten dargestellt, die ersten drei Schritte 23, 24 und 25 wie in der Erläuterung zu Fig. 2 beschrieben, erfolgen, dann aber im vierten Schritt 30 die Fokuspunkte vom vierten chromatischen Tiefenmessbereich 15.4 zum dritten chromatischen Tiefenmessbereich 15.3 verschoben werden, nach der Aufnahme des dritten Tiefenmessbereichs 15.3 in einem fünften Schritt 31 zum zweiten Tiefenmessbereich 15.2 verschoben werden und nach der Aufnahme des zweiten Tiefenmessbereichs 15.2 in einem letzten sechsten Schritt 32 zum ersten Tiefenmessbereich 15.1 verschoben werden. Die Verstellung der chromatischen Tiefenmessbereiche in den ersten drei Schritten 23, 24 und 25 erfolgt dabei zur Vermessung eines Messpunktes 17 aus Fig. 1, wobei anschließend der Beleuchtungsstrahl 8 durch Verkippen des schwenkbaren Spiegels 5 in seitlicher Richtung verstellt wird, so dass die Verstellung zwischen den chromatischen Tiefenmessbereichen im vierten, fünften und sechsten Schritt 30, 31 und 32 zur Vermessung eines benachbarten Messpunktes 17' aus Fig. 1 erfolgt. Dieser Messzyklus des Beleuchtungsstrahls wird so lange wiederholt, bis die Vermessung der Oberfläche 10 abgeschlossen ist.

### Bezugszeichenliste

- 1: handgehaltene dentale Kamera
- 2: Objektiv
- 3: Lichtquelle
- 4: Farbsensor
- 5: schwenkbarer Spiegel
- 5.1: Stellung
- 5.2: Stellung
- 6: Strahlteiler
- 7: Umlenkmittel
- 8: Beleuchtungsstrahl
- 8': Beobachtungsstrahl
- 9: Messobjekt
- 10: Oberfläche
- 11: Anschluss
- 12: Datenverarbeitungseinheit
- 13: Fokuspunkt
- 14: Fokuspunkt
- 15.1: chromatischer Tiefenmessbereich
- 15.2: chromatischer Tiefenmessbereich
- 15.3: chromatischer Tiefenmessbereich
- 15.4: chromatischer Tiefenmessbereich
- 16: gesamter Tiefenmessbereich
- 17: Messpunkt
- 17': Messpunkt
- 18: Abstand
- 19: Anzeigeeinrichtung
- 20: Scaneinheit
- 20': Scaneinheit in einer zweiten Stellung
- 21: Bewegungsrichtung der Scaneinheit
- 23: Schritt
- 24: Schritt
- 25: Schritt
- 26: Schritt
- 30: Schritt
- 31: Schritt
- 32: Schritt

## Patentansprüche

1. Handgehaltene dentale Kamera (1) zur optischen 3D-Vermessung, umfassend ein chromatisches Objektiv (2), eine polychromatische Lichtquelle (3) und einen Farbsensor (4), wobei die polychromatische Lichtquelle (3) einen Beleuchtungsstrahl (8) abstrahlt, der mittels des chromatischen Objektivs (2) auf eine Oberfläche (10) eines Messobjekts (9) zumindest bezüglich einer Wellenlänge fokussierbar ist, wobei der Beleuchtungsstrahl (8) von der Oberfläche (10) als ein Beobachtungsstrahl (8') zurückgestrahlt und mittels des Farbsensors (4) detektierbar ist, wobei die Fokuspunkte (13, 14) verschiedener Wellenlängen des Beleuchtungsstrahls (8) einen chromatischen Tiefenmessbereich (15.1, 15.2, 15.3, 15.4) bilden und wobei die handgehaltene dentale Kamera (1) eine entlang einer Längsachse (A) verstellbare Scaneinheit (20) aufweist, die zumindest das chromatische Objektiv (2) umfasst, **dadurch gekennzeichnet, dass** die Scaneinheit (20) in genau zwei mit einem Endanschlag realisierten Stellungen von der einen Endstellung unmittelbar in die andere Endstellung bringbar ist und dass der chromatische Tiefenmessbereich (15.1, 15.2, 15.3, 15.4) mittels der Scaneinheit (20) schrittweise mit einer Schrittweite kleiner oder gleich der Länge des chromatischen Tiefenmessbereichs so verschiebbar ist, dass an einen ersten chromatischen Tiefenmessbereich (15.1) in der einen Endstellung der Scaneinheit (20) in Richtung der Messtiefe genau ein zweiter chromatischer Tiefenmessbereich (15.2, 15.3, 15.4) in der anderen Endstellung der Scaneinheit (20') anschließt oder sich mit dem ersten chromatischen Tiefenmessbereich (15.1) teilweise überlappt, dass die handgehaltene dentale Kamera (1) ein Umlenkmittel (7) umfasst, das nicht Teil der Scaneinheit (20) ist, wobei das Umlenkmittel (7) zwischen dem chromatischen Objektiv (2) und dem Messobjekt (9) angeordnet ist, wobei der Beleuchtungsstrahl (8) mittels des Umlenkmittels (7) quer zur Längsachse (A) der handgehaltenen dentalen Kamera (1) zum Messobjekt (9) hin umlenkbar ist.

2. Handgehaltene dentale Kamera (1) nach Anspruch 1, **dadurch gekennzeichnet, dass** die Lichtquelle (3) eine Halogen- oder Xenon-Gasentladungslampe mit einem Strahlungsspektrum von 300 nm bis 2000 nm ist.

3. Handgehaltene dentale Kamera (1) nach Anspruch 1, **dadurch gekennzeichnet, dass** die Lichtquelle (3) eine Superlumineszenzdiode (SLD) ist.

4. Handgehaltene dentale Kamera (1) nach Anspruch 3, **dadurch gekennzeichnet, dass** die Superlumineszenzdiode (SLD) ein Strahlungsspektrum von 900 nm bis 1000 nm aufweist.

5. Handgehaltene dentale Kamera (1) nach Anspruch 3, **dadurch gekennzeichnet, dass** die Superlumineszenzdiode (SLD) ein Strahlungsspektrum von 1500 nm bis 1650 nm aufweist.

6. Handgehaltene dentale Kamera (1) nach Anspruch 1, **dadurch gekennzeichnet, dass** die Lichtquelle (3) eine Weißlicht LED ist.

7. Handgehaltene dentale Kamera (1) nach einem der Ansprüche 1 bis 6, **dadurch gekennzeichnet, dass** der chromatische Tiefenmessbereich (15.1, 15.2, 15.3, 15.4) eine Länge zwischen 0,5 mm und 5 mm aufweist.

8. Handgehaltene dentale Kamera (1) nach einem der Ansprüche 1 bis 7, **dadurch gekennzeichnet, dass** die handgehaltene dentale Kamera (1) eine Datenverarbeitungseinheit (12) aufweist oder einen Anschluss aufweist, an den eine Datenverarbeitungseinheit (12) anschließbar ist, wobei für jeden chromatischen Tiefenmessbereichen (15.1, 15.2, 15.3, 15.4) ein Datensatz aufnehmbar und in der Datenverarbeitungseinheit (12) speicherbar ist und diese verschiedenen Datensätze zu einem gesamten 3D-Datensatz über den gesamten Tiefenmessbereich (16) zusammenfügbar sind.

9. Handgehaltene dentale Kamera (1) nach einem der Ansprüche 1 bis 8, **dadurch gekennzeichnet, dass** die Scaneinheit (20) mit einer Frequenz zwischen 1Hz und 1000Hz mechanische verstellbar ist.

10. Handgehaltene dentale Kamera (1) nach einem der Ansprüche 1 bis 9, **dadurch gekennzeichnet, dass** ein um mindestens eine Achse quer zur Längschse (A) der dentalen Kamera (1) schwenkbarer Spiegel (5) zwischen der Lichtquelle (3) und der Oberfläche (10) des Messobjekts (9) angeordnet ist, so dass durch ein schrittweises Verkippen des schwenkbaren Spiegels (5) der Beleuchtungsstrahl (8) schrittweise quer zur Längsachse (A) der dentalen Kamera (1) bewegbar ist.

11. Verfahren zur optischen 3D-Vermessung, wobei ein Beleuchtungsstrahl (8) einer polychromatischen Lichtquelle (3), der durch ein chromatisches Objektiv (2) auf eine Oberfläche (10) eines Messobjekts (9) zumindest bezüglich einer Wellenlänge fokussiert wird und der von der Oberfläche (10) als ein Beobachtungsstrahl (8') zurückgestrahlte Beleuchtungsstrahl (8) mit einem Farbsensor (4) detektiert wird, wobei die Fokuspunkte (13, 14) verschiedener Wellenlängen des Beleuchtungsstrahls (8) einen chromatischen Tiefenmessbereich (15.1, 15.2, 15.3, 15.4) bilden, **dadurch gekennzeichnet, dass** eine Scaneinheit (20), die zumindest das chromatische Objektiv (2) umfasst, schrittweise mit einer Schrittweite kleiner oder gleich der Länge des chromatischen Tiefenmessbereichs entlang einer Längsachse (A) verschoben wird, dass die Scaneinheit (20) genau einmal von einer mit einem Endanschlag realisierten Endstellung unmittelbar in eine andere mit einem Endanschlag realisierten Endstellung verschoben wird, so dass an einen ersten chromatischen Tiefenmessbereich (15.1) in der einen Endstellung der Scaneinheit (20) in Richtung einer Messtiefe genau ein zweiter chromatischer Tiefenmessbereich(15.2, 15.3, 15.4) in der anderen Endstellung der Scaneinheit angeschlossen wird oder mit dem ersten chromatischen Tiefenmessbereich (15.1) teilweise überlappt wird und dass der Beleuchtungsstrahl (8) mittels eines Umlenkmittels (7), das nicht Teil der Scaneinheit (20) ist, zum Messobjekt (9) hin quer zur Längsachse (A) der handgehaltenen dentalen Kamera (1) umgelenkt wird.

12. Verfahren nach Anspruch 11, **dadurch gekennzeichnet, dass** eine Lichtquelle (3) mit eine Spektralbereich von 300nm bis 2000nm verwendet wird.

13. Verfahren nach einem der Ansprüche 11 bis 12, **dadurch gekennzeichnet, dass** eine Lichtquelle (3) mit einem Spektralbereich von 900nm bis 1000nm oder von 1500nm bis 1650nm verwendet wird.

14. Verfahren nach einem der Ansprüche 11 bis 13, **dadurch gekennzeichnet, dass** Fokuspunkte (13, 14) für verschiedene Wellenlängen aufgefächert werden, so dass sich eine Länge des chromatischen Tiefenmessbereichs (15.1, 15.2, 15.3, 15.4) zwischen 0,5mm und 5mm ergibt.

15. Verfahren nach einem der Ansprüche 11 bis 14, **dadurch gekennzeichnet, dass** für jeden chromatischen Tiefenmessbereich (15.1, 15.2, 15.3, 15.4) ein Datensatz aufgenommen wird, wobei die Datensätze innerhalb der Kamera (1) gespeichert und zu einem 3D-Datensatz des Messobjekts (2) zusammengefügt oder an eine Datenverarbeitungseinheit (12) übermittelt werden.

16. Verfahren nach einem der Ansprüche 11 bis 15, **dadurch gekennzeichnet, dass** die Scaneinheit (20) mit einer Frequenz zwischen 1 Hz und 1000 Hz verschoben wird.

17. Verfahren nach einem der Ansprüche 11 bis 16, **dadurch gekennzeichnet, dass** der Beleuchtungsstrahl (8) durch schrittweises Verkippen eines um mindestens eine Achse quer zur Längschse (A) der dentalen Kamera (1) schwenkbaren Spiegels (5) quer zur Längsachse (A) der dentalen Kamera (1) bewegt wird.

18. Verfahren nach einem der Ansprüche 11 bis 17, **dadurch gekennzeichnet, dass** das Verschieben der Scaneinheit (20) zum Vermessen der einzelnen chromatischen Messbereiche (15.1, 15.2, 15.3, 15.4), die den gesamten Messbereich (16) ergeben, in mehreren Schritten (23, 24, 25) erfolgt und anschließend die Scaneinheit (20) in einem weiteren Schritt (26) wieder in die erste Stellung gebracht wird, wobei dieser Messzyklus der Scaneinheit (20) solange wiederholt wird, bis die Messung abgeschlossen ist.

19. Verfahren nach einem der Ansprüche 11 bis 17, **dadurch gekennzeichnet, dass** das Verschieben der Scaneinheit (20) zum Vermessen der einzelnen chromatischen Messbereiche (15.1, 15.2, 15.3, 15.4), die den gesamten Messbereich (16) ergeben, in mehreren Schritten (23, 24, 25) erfolgt und anschließend die Scaneinheit (20) wieder in Schritten (30, 31, 32) in die erste Stellung gebracht wird, wobei dieser Messzyklus der Scaneinheit solange wiederholt wird, bis die Messung abgeschlossen ist.

## Claims

1. A handheld dental camera (1) for carrying out optical 3D measurement, comprising a chromatic objective (2), a polychromatic light source (3), and a color sensor (4), wherein said polychromatic light source (3) emits an illuminating beam (8), which is capable of being focused, in terms of at least one wavelength thereof, by means of said chromatic objective (2) onto a surface (10) of an object to be measured (9) and said illuminating beam (8) is reflected by said surface (10) to form a monitoring beam (8') and is capable of being detected by means of said color sensor (4), and the focal points (13 14) of the various wavelengths of said illuminating beam (8) form a chromatic depth measurement range (15.1, 15.2, 15.3, 15.4) and said handheld dental camera (1) comprises a scanning unit (20) which is capable of being moved along a longitudinal axis (A) and which comprises at least said chromatic objective (2), **characterized in that** said scanning unit (20) is capable of being moved to exactly two positions defined by end stops, namely from one end position directly to the other end position, and said chromatic depth measurement range (15.1, 15.2, 15.3, 15.4) is capable of being displaced stepwise by a step width which is smaller than or equal to the length of the chromatic depth measurement range, by means of said scanning unit (20), such that a first chromatic depth measurement range (15.1) in one end position (20) of said scanning unit is, in the direction of the measurement depth, precisely adjoined, or partially overlapped, by at least a second chromatic depth measurement range (15.2, 15.3, 15.4) in the other end position (20') of said scanning unit, and said handheld dental camera (1) comprises a deflector (7), which is not part of said scanning unit (2), which deflector (7) is disposed between (7) said chromatic objective (2) and said object (9) to be measured, and the illuminating beam (8) is capable of being deflected by said deflector (7) in a direction at right angles to said longitudinal axis (A) of said handheld dental camera (1), toward said object (9) to be measured.

2. The handheld dental camera (1) as defined in claim 1, **characterized in that** that said light source (3) is a halogen lamp or a xenon gas discharge lamp having a radiation spectrum of from 300 nm to 2000 nm.

3. The handheld dental camera (1) as defined in claim 1, **characterized in that** said light source (3) is a superluminescent diode (SLD).

4. The handheld dental camera (1) as defined in claim 3, **characterized in that** said superluminescent diode (SLD) has a radiation spectrum of from 900 nm to 1000 nm.

5. The handheld dental camera (1) as defined in claim 3, **characterized in that** said superluminescent diode (SLD) has a radiation spectrum of from 1500 nm to 1650 nm.

6. The handheld dental camera (1) as defined in claim 1, **characterized in that** said light source (3) is a white light LED.

7. The handheld dental camera (1) as defined in any one of claims 1 to 6, **characterized in that** said chromatic depth measurement range (15.1, 15.2, 15.3, 15.4) has a length of from 0.5 mm to 5 mm.

8. The handheld dental camera (1) as defined in any one of claims 1 to 7, **characterized in that** said handheld dental camera (1) has a data processing unit (12) or has a connector to which a data processing unit (12) can be connected, and for each chromatic depth measurement range (15.1, 15.2, 15.3, 15.4) a data set can be acquired and saved in said data processing unit (12), and the various data sets can be combined to form an overall 3D data set covering the total depth measurement range (16).

9. The handheld dental camera (1) as defined in any one of claims 1 to 8, **characterized in that** said scanning unit (20) can be mechanically moved at a frequency of from 1 Hz to 1000 Hz.

10. The handheld dental camera (1) as defined in any one of claims 1 to 9, **characterized in that** a mirror (5), which is pivotal about at least an axis at right angles to the longitudinal axis (A) of said dental camera (1), is disposed between said light source (3) and the surface (10) of said object to be measured (9), such that the illuminating beam (8) can be moved stepwise in a direction at right angles to said longitudinal axis (A) of said dental camera (1) by stepwise tilting of said pivotal mirror (5).

11. A method for carrying out optical 3D measurement, wherein an illuminating beam (8) that is emitted by a polychromatic light source and focused, in terms of at least one wavelength thereof, by means of a chromatic objective (2) onto a surface (10) of an object to be measured (9) and which is reflected by said surface (10) to form a monitoring beam (8') and is detected by means of a color sensor (4), and the focal points (13, 14) of the various wavelengths of said illuminating beam (8) form a chromatic depth measurement range (15.1, 15.2, 15.3, 15.4), **characterized in that** the scanning unit (20) is moved to exactly two positions defined by end stops, namely from one end position directly to the other end position, and said chromatic depth measurement range (15.1, 15.2, 15.3, 15.4) is displaced stepwise by a step width that is smaller than or equal to the length of the chromatic depth measurement range, by means of said scanning unit (20), such that a first chromatic depth measurement range (15.1) in one end position of said scanning unit (20) is, in the direction of the measurement depth, precisely adjoined, or partially overlapped, by at least a second chromatic depth measurement range (15.2, 15.3, 15.4) in the other end position of said scanning unit, and said handheld dental camera (1) comprises a deflector (7), which is not part of said scanning unit (2), which deflector (7) is disposed between (7) said chromatic objective (2) and said object (9) to be measured, and said illuminating beam (8) is deflected by said deflector (7) in a direction at right angles to said longitudinal axis (A) of said handheld dental camera (1) toward said object (9) to be measured.

12. The method as defined in any one of claims 11, **characterized in that** a light source (3) is used that has a spectral range of from 300 nm to 2000 nm.

13. The method as defined in any one of claim 11 and claim 12, **characterized in that** a light source (3) is used that has a spectral range of from 900 nm to 1000 nm or from 1500 nm to 1650 nm.

14. The method as defined in any one of claims 11 to 13, **characterized in that** the focal points (13 14) for the various wavelengths are fanned out such that the length of a chromatic depth measurement range (15.1, 15.2, 15.3, 15.4) is from 0.5 mm to 5 mm.

15. The method as defined in any one of claims 11 to 14, **characterized in that** for each chromatic depth measurement range (15.1, 15.2, 15.3, 15.4) a data set is acquired and said data sets are saved within the camera (1) and are then combined to form a 3D data set of said object to be measured (2) or are transferred to a data processing unit (12).

16. The method as defined in any one of claims 11 to 15, **characterized in that** said scanning unit (20) is moved at a frequency of from 1 Hz to 1000 Hz.

17. The method as defined in any one of claims 11 to 16, **characterized in that** said illuminating beam (8) is moved stepwise in a direction at right angles to said longitudinal axis (A) of said dental camera (1) by stepwise tilting of said pivotal mirror (5).

18. The method as defined in any one of claims 11 to 17, **characterized in that** the movement of said scanning unit (20) for the purpose of measuring the individual chromatic measurement ranges (15.1, 15.2, 15.3, 15.4) giving the overall measurement range (16) is carried out in a plurality of steps (23, 24, 25), and the scanning unit (20) is then moved back to its initial position in a further step (26), and this measuring cycle of the scanning unit (20) is repeated until measurement is complete.

19. The method as defined in any one of claims 11 to 17, **characterized in that** the movement of said scanning unit (20) for the purpose of measuring the individual chromatic measurement ranges (15.1, 15.2, 15.3, 15.4) giving the overall measurement range (16) is carried out in a plurality of steps (23, 24, 25), and the scanning unit (20) is then moved back to its initial position in a plurality of steps (23, 24, 25), and this measuring cycle of the scanning unit (20) is repeated until measurement is complete.

## Revendications

1. Caméra dentaire portative (1) pour prise de mesure 3D optique, comprenant un objectif chromatique (2), une source de lumière polychromatique (3) et un capteur de couleur (4), dans laquelle la source de lumière polychromatique (3) émet un rayon d'éclairage (8) qui peut être focalisé au moyen de l'objectif chromatique (2) sur une surface (10) d'un objet mesuré (9) au moins par rapport à une longueur d'onde, dans laquelle le rayon d'éclairage (8) est renvoyé par la surface (10) sous forme de rayon d'observation (8') et décelable au moyen du capteur de couleur (4), dans laquelle les points focaux (13, 14) de différentes longueurs d'onde du rayon d'éclairage (8) constituent une plage de mesure de profondeur chromatique (15.1, 15.2, 15.3, 15.4), et dans laquelle la caméra dentaire portative (1) présente une unité de balayage (20) réglable suivant un axe longitudinal (A) et comprenant au moins l'objectif chromatique (2), **caractérisée en ce que** l'unité de balayage (20) peut être amenée dans exactement deux positions matérialisées par une butée de fin de course, de ladite une position d'extrémité directement à l'autre position d'extrémité, et **en ce que** la plage de mesure de profondeur chromatique (15.1, 15.2, 15.3, 15.4) peut être décalée progressivement au moyen de l'unité de balayage (20) avec un pas de progression inférieur ou égal à la longueur de la plage de mesure de profondeur chromatique de telle sorte qu'une première plage de mesure de profondeur chromatique (15.1) à ladite une position d'extrémité de l'unité de balayage (20) est suivie en direction de la profondeur de mesure d'exactement une deuxième plage de mesure de profondeur chromatique (15.2, 15.3, 15.4) à l'autre position d'extrémité de l'unité de balayage (20') ou que celle-ci recouvre partiellement la première plage de mesure de profondeur chromatique (15.1), **en ce que** la caméra dentaire portative (1) comprend un moyen de déviation (7) qui ne fait pas partie de l'unité de balayage (20), dans laquelle le moyen de déviation (7) est disposé entre l'objectif chromatique (2) et l'objet mesuré (9), dans laquelle le rayon d'éclairage (8) peut être dévié à l'aide du moyen de déviation (7) transversalement à l'axe longitudinal (A) de la caméra dentaire portative (1) vers l'objet mesuré (9).

2. Caméra dentaire portative (1) selon la revendication 1, **caractérisée en ce que** la source de lumière (3) est une lampe halogène ou à décharge au xénon avec un spectre de radiation de 300 nm à 2000 nm.

3. Caméra dentaire portative (1) selon la revendication 1, **caractérisée en ce que** la source de lumière (3) est une diode superluminescente (SLD).

4. Caméra dentaire portative (1) selon la revendication 3, **caractérisée en ce que** la diode superluminescente (SLD) présente un spectre de radiation de 900 nm à 1000 nm.

5. Caméra dentaire portative (1) selon la revendication 3, **caractérisée en ce que** la diode superluminescente (SLD) présente un spectre de radiation de 1500 nm à 1650 nm.

6. Caméra dentaire portative (1) selon la revendication 1, **caractérisée en ce que** la source de lumière (3) est une LED à lumière blanche.

7. Caméra dentaire portative (1) selon l'une quelconque des revendications 1 à 6, **caractérisée en ce que** la plage de mesure de profondeur chromatique (15.1, 15.2, 15.3, 15.4) présente une longueur entre 0,5 mm et 5 mm.

8. Caméra dentaire portative (1) selon l'une quelconque des revendications 1 à 7, **caractérisée en ce que** la caméra dentaire portative (1) présente une unité de traitement de données (12) ou un connecteur sur lequel on peut brancher une unité de traitement de données (12), dans laquelle pour chaque plage de mesure de profondeur chromatique (15.1, 15.2, 15.3, 15.4), un ensemble de données peut être enregistré et stocké dans l'unité de traitement de données (12), et ces divers ensembles de données peuvent être assemblés en un ensemble de données 3D global sur toute la plage de mesure de profondeur (16).

9. Caméra dentaire portative (1) selon l'une quelconque des revendications 1 à 8, **caractérisée en ce que** l'unité de balayage (20) est réglable mécaniquement sur une fréquence entre 1 Hz et 1000 Hz.

10. Caméra dentaire portative (1) selon l'une quelconque des revendications 1 à 9, **caractérisée en ce qu'**un miroir (5) pivotant autour d'au moins un axe transversal à l'axe longitudinal (A) de la caméra dentaire (1) est disposé entre la source de lumière (3) et la surface (10) de l'objet mesuré (9), de sorte qu'un basculement progressif du miroir pivotant (5) permet de déplacer progressivement le rayon d'éclairage (8) transversalement à l'axe longitudinal (A) de la caméra dentaire (1).

11. Procédé de prise de mesures 3D optique, dans lequel un rayon d'éclairage (8) d'une source de lumière polychromatique (3) est focalisé par un objectif chromatique (2) sur une surface (10) d'un objet mesuré (9) au moins par rapport à une longueur d'onde, et est détecté par un capteur de couleur (4) sous la forme d'un rayon d'éclairage (8) renvoyé par la surface (10) sous la forme d'un rayon d'observation (8'), dans lequel les points focaux (13, 14) de différentes longueurs d'onde du rayon d'éclairage (8) constituent une plage de mesure de profondeur chromatique (15.1, 15.2, 15.3, 15.4), **caractérisé en ce qu'**une unité de balayage (20) qui comprend au moins l'objectif chromatique (2) est décalée progressivement avec un pas de progression inférieur ou égal à la longueur de la plage de mesure de profondeur chromatique suivant un axe longitudinal (A), **en ce que** l'unité de balayage (20) est décalée exactement une fois d'une position d'extrémité matérialisée par une butée de fin de course directement à une autre position d'extrémité matérialisée par une butée de fin de course, de sorte qu'une première plage de mesure de profondeur chromatique (15.1) à ladite une position d'extrémité de l'unité de balayage (20) est suivie en direction de la profondeur de mesure d'exactement une deuxième plage de mesure de profondeur chromatique (15.2, 15.3, 15.4) à l'autre position d'extrémité de l'unité de balayage ou que celle-ci recouvre partiellement la première plage de mesure de profondeur chromatique (15.1), et **en ce que** le rayon d'éclairage (8) est dévié à l'aide d'un moyen de déviation (7) qui ne fait pas partie de l'unité de balayage (20) vers l'objet mesuré (9) transversalement à l'axe longitudinal (A) de la caméra dentaire portative (1) .

12. Procédé selon la revendication 11, **caractérisé en ce qu'**une source de lumière (3) avec un domaine spectral de 300 nm à 2000 nm est utilisée.

13. Procédé selon l'une quelconque des revendications 11 à 12, **caractérisé en ce qu'**une source de lumière (3) avec un domaine spectral de 900 nm à 1000 nm ou de 1500 nm à 1650 nm est utilisée.

14. Procédé selon l'une quelconque des revendications 11 à 13, **caractérisé en ce que** des points focaux (13, 14) sont ouverts en éventail pour différentes longueurs d'onde de sorte qu'il en résulte une longueur de la plage de mesure de profondeur chromatique (15.1, 15.2, 15.3, 15.4) entre 0,5 mm et 5 mm.

15. Procédé selon l'une quelconque des revendications 11 à 14, **caractérisé en ce que** pour chaque plage de mesure de profondeur chromatique (15.1, 15.2, 15.3, 15.4), un ensemble de données est enregistré, dans lequel les ensembles de données sont stockés à l'intérieur de la caméra (1) et assemblés en un ensemble de données 3D de l'objet mesuré (2) ou transmis à une unité de traitement de données (12).

16. Procédé selon l'une quelconque des revendications 11 à 15, **caractérisé en ce que** l'unité de balayage (20) est décalée à une fréquence entre 1 Hz et 1000 Hz.

17. Procédé selon l'une quelconque des revendications 11 à 16, **caractérisé en ce que** le rayon d'éclairage (8) est déplacé par basculement progressif d'un miroir (5) pivotant autour d'au moins un axe transversal à l'axe longitudinal (A) de la caméra dentaire (1), transversalement à l'axe longitudinal (A) de la caméra dentaire (1).

18. Procédé selon l'une quelconque des revendications 11 à 17, **caractérisé en ce que** le décalage de l'unité de balayage (20) en vue d'une prise de mesures des zones de mesure chromatiques individuelles (15.1, 15.2, 15.3, 15.4) qui constituent l'ensemble de la plage de mesure (16) est effectué en plusieurs étapes (23, 24, 25), et qu'ensuite, dans une étape (26) supplémentaire, l'unité de balayage (20) est ramenée à la première position, ce cycle de mesure de l'unité de balayage (20) étant répété jusqu'à ce que la mesure soit terminée.

19. Procédé selon l'une quelconque des revendications 11 à 17, **caractérisé en ce que** le décalage de l'unité de balayage (20) en vue d'une prise de mesures des zones de mesure chromatiques individuelles (15.1, 15.2, 15.3, 15.4) qui constituent l'ensemble de la plage de mesure (16) est effectué en plusieurs étapes (23, 24, 25), et qu'ensuite l'unité de balayage (20) est ramenée pas à pas (30, 31, 32) à la première position, ledit cycle de mesure de l'unité de balayage étant répété jusqu'à ce que la mesure soit terminée.
